# EUROPÄISCHE PATENTSCHRIFT

(11) **EP 2 389 432 B1**
(45) Veröffentlichungstag und Bekanntmachung des Hinweises auf die Patenterteilung: **28.11.2012**
(21) Anmeldenummer: 10725374.2
(22) Anmeldetag: 21.01.2010
(51) Int. Cl.: C12M 1/107

(54) **BIOGASANLAGEN-SERVICEEINRICHTUNG**
BIOGAS PLANT SERVICE DEVICE
SYSTÈME DE SERVICE POUR INSTALLATION DE PRODUCTION DE BIOGAZ

(30) Priorität: 21.01.2009 DE 102009005628; 20.07.2009 DE 102009034127
(43) Veröffentlichungstag der Anmeldung: 30.11.2011
(73) Patentinhaber: Uts Biogastechnik GmbH, 85399 Hallbergmoos (DE)
(72) Erfinder: BIERER, Johann, 84405 Dorfen (DE); RABENER, Matthias, 59302 Oelde (DE); CZWALUK, Andreas, 49377 Vechta (DE)
(74) Vertreter: Schütte, Hartmut
(86) Internationale Anmeldenummer: PCT/EP2010/000346
(87) Internationale Veröffentlichungsnummer: WO 2010/084003

(56) Entgegenhaltungen:
- EP-B1- 1 717 305
- DE-C1- 4 015 478
- DE-U1-202004 004 101

## Beschreibung

Die vorliegende Erfindung betrifft eine Biogasanlagen-Serviceeinrichtung mit wenigstens einem Serviceschacht für wenigstens einen Fermenterbehälter einer Biogasanlage. Der Serviceschacht ist insbesondere an einem den Fermenterbehälter der Biogasanlage abdeckenden Behälterdach angeordnet. Das Behälterdach ist vorzugsweise gegen die Horizontale unter einem Winkel Alpha geneigt. Möglich ist aber auch der Einsatz an Flachdächern oder im Wesentlichen flach ausgebildeten Dächern. Der Serviceschacht ist dabei insbesondere in einer das Behälterdach abdichtenden Weise ausgebildet und unter einer Neigung angeordnet, die der Neigung des Behälterdaches entspricht.

Aus der EP 1 717 305 B1 geht eine Biogasanlagen-Serviceeinrichtung bzw. ein Serviceschacht für einen Fermenterbehälter einer Biogasanlage hervor, wobei der Serviceschacht als gasdichte Abdeckung domartig über einer in einer Fermenter-Behälterabdeckung bzw. in einem Behälterdach ausgebildeten Serviceöffnung angebracht ist, durch die hindurch eine Rühreinheit geht, die an einer das Innere des Fermenterbehälters durchdringenden Montagestange höhenverstellbar geführt und gehalten ist. Darüber hinaus kann die Rühreinheit aus dem Fermenterbehärter heraus in den Serviceschacht-Innenraum verbracht werden. Der Innenraum des Serviceschachtes hat eine verschließbare Zugangsöffnung. Das Behälterdach ist als ein gegen die Horizontale geneigtes Behälterdach ausgebildet, durch das der Serviceschacht mit einem unteren Schachtende unter Ausbildung der Serviceöffnung gasdicht hindurch und in den FermenterBehälterinnenraum geführt ist, in dem das untere Schachtende an einer eine horizontale Anschlussebene aufweisenden und im Behälterinnenraum angeordneten Tragkonsole abgestützt und festgelegt ist. Diese wiederum ist über Diagonalstreben an der Innenseite der Wand des Fermenterbehälters abgestützt und dort verankert.

Dieser bekannte Stand der Technik funktioniert und erfüllt seinen Zweck auf zufriedenstellende Weise. Es ist die Aufgabe der vorliegenden Erfindung einen anderen Serviceschacht oder eine andere Serviceeinrichtung zur Verfügung zu stellen, welche unabhängig von dem angeführten Patent eine zufriedenstellende Funktion ermöglicht.

Diese Aufgabe wird gelöst durch die Biogasanlagen-Serviceeinrichtung mit den Merkmalen des Anspruchs 1 und durch eine Biogasanlage mit den Merkmalen des Anspruchs 15. Bevorzugte Weiterbildungen der Erfindung sind Gegenstand der Unteransprüche. Weitere Vorteile und Merkmale der Erfindung ergeben sich aus den Ausführungsbeispielen.

Die erfindungsgemäße Biogasanlagen-Serviceeinrichtung mit wenigstens einem Serviceschacht ist für den Einsatz an einer Biogasanlage vorgesehen. Eine dafür geeignete Biogasanlage weist wenigstens einen Fermenterbehälter mit wenigstens einer äußeren Behälterwand und wenigstens einen Fermenterbehälterinnenraum und wenigstens ein den Fermenterbehälterinnenraum abdeckendes Behälterdach auf. Der Serviceschacht der Biogasanlagen-Serviceeinrichtung weist wenigstens eine Serviceöffnung auf, um insbesondere den Ein- und Ausbau und/oder die Wartung von Geräten, wie z.B. eines Rührgerätes zu erleichtern. Der Serviceschacht ist an einem den Fermenterbehälterinnenraum wenigstens teilweise abdeckenden Behälterdach in einer das Behälterdach abdichtenden Weise anzuordnen und insbesondere an einem solchen angeordnet. Dabei ist wenigstens eine in den Fermenterbehälterinnenraum und insbesondere in die Fermentermasse hineinragende und von dem Serviceschacht ausgehende bzw. sich von dort in den Fermenterbehälter erstreckende Montagestange vorgesehen. Die Montagestange ist von der Behälterwand beabstandet anzuordnen bzw. anordnenbar. Die Montagestange ist insbesondere beabstandet von der Behälterwand aufstellbar oder befestigbar. Der Serviceschacht ist an wenigstens einem Traggestell befestigt, welches wenigstens einen Tragabschnitt umfasst. An dem Tragabschnitt sind Befestigungsmittel vorgesehen, mit denen der Tragabschnitt ausrichtbar und außerhalb des Fermenterbehälterinnenraums befestigbar ist.

Die erfindungsgemäße Biogasanlagen-Serviceeinrichtung hat viele Vorteile. Ein erheblicher Vorteil ist, dass der Tragabschnitt nicht das Behälterdach durchsetzt. Eine Befestigung an der inneren Behälterwand im Innenraum des Fermenterbehälters ist nicht nötig. Dadurch wird ein erheblicher Aufwand eingespart.

Gegenüber dem vorbekannten Stand der Technik hat die Erfindung auch den Vorteil, dass die gesamte Halterung des Serviceschachtes nicht der aggressiven Umgebung innerhalb des Fermenterbehälters ausgesetzt ist. Die tragende Konstruktion kommt mit der Fermentermasse insbesondere nicht in dauerhaften Kontakt. Dadurch können einfachere und günstigere Materialien zur Konstruktion verwendet werden. Ein weiterer erheblicher Vorteil ist, dass die Strömung der Fermentermasse nicht durch die Tragkonstruktion beeinträchtigt wird.

Weiterhin ist im Stand der Technik die Halterung an der Innenseite der Wand des Fermenterbehälters im Innenraum des Fermenterbehälters komplizierter und gegebenenfalls auch wegen der aggressiven Umgebung störanfälliger.

Insbesondere kann der Serviceschacht wenigstens eine zum Serviceschacht hinführende Arbeitsplattform umfassen. Das Traggestell kann als Konsole ausgebildet sein, an dem der Serviceschacht befestigt ist. Dann kann die Konsole wenigstens einen Tragabschnitt und wenigstens eine zum Serviceschacht hinführende oder den Serviceschacht umfassende Arbeitsplattform aufweisen.

In allen Ausgestaltungen kann die Befestigung des Serviceschachtes oben an der Behälterwand oder radial außen an der Außenseite der Behälterwand erfolgen. Eine Befestigung radial an der inneren Behälterwand innerhalb des Innenraums ist nicht nötig.

Eine Befestigung auf der Innenseite der Behälterwand ist erfindungsgemäß aber auch oberhalb des Behälterdaches auf der Innenseite der Behälterwand möglich. Beispielsweise dann, wenn die Behälterwand über das Behälterdach nach oben hin übersteht. Dann ist eine Befestigung des Serviceschachtes oberhalb des Behälterdaches auch an der Innenseite der Behälterwand möglich, ohne dass der Tragabschnitt in dauerhaftem Kontakt mit der Fermentermasse oder dem erzeugten Biogas steht. In bevorzugten Ausgestaltungen wird der Tragabschnitt außerhalb des Fermenterbehälterinnenraums an der Behälterwand befestigt. Der Tragabschnitt kann an der Außenseite der Behälterwand und/oder an dem oberen Ende der Behälterwand und/oder oberhalb des Behälterdaches an der Innenseite der Behälterwand befestigt werden.

Der Tragabschnitt kann dazu ausgebildet und geeignet sein, an seinem unteren Endbereich in ein Fundament eingelassen zu werden. Insbesondere ist der Tragabschnitt dazu ausgebildet, an oder in der Nähe der Außenseite der Behälterwand in ein Fundament eingelassen zu werden, welches mit dem Fundament des Fermenterbehälters verbunden oder separat ausgebildet sein kann.

Vorteilhaftweise ist das Traggestell bzw. die Konsole insgesamt so ausgebildet, dass die Arbeitsplattform und der Tragabschnitt über wenigstens eine Verstärkungsstrebe miteinander verbunden sind. Dadurch wird gewährleistet, dass die Konsole über eine genügende Eigensteifigkeit verfügt, um z.B. den zu einer vorbestimmten Stelle des Behälterdaches ausgerichteten Serviceschacht tragen zu können.

Die Arbeitsplattform ist vorzugsweise im Wesentlichen horizontal ausrichtbar bzw. ausgerichtet. Eine Neigung des Tragabschnitts entspricht im Wesentlichen einer Neigung der äußeren Behälterwand. Vorzugsweise ist der Tragabschnitt wenigstens abschnittsweise etwa senkrecht angeordnet.

Vorzugsweise ist das Behälterdach wenigstens teilweise als Foliendach ausgebildet oder umfasst wenigstens ein Foliendach. Insbesondere bei Foliendächern kann die bekannte Art der Tragekonstruktion des Serviceschachtes von der Unterseite des Behälter- bzw. Foliendaches her an ihre Grenzen stoßen, wenn der Neigungswinkel des Behälterdaches bzw. des Foliendaches vergrößert wird. Bei großen Steigungen des Foliendaches wird eine Anbringung des Serviceschachtes an der Innenseite der Behälterwand aufwändig. Demgegenüber bietet die erfindungsgemäße Konstruktion erhebliche konstruktive Freiräume.

Besonders vorteilhaft ist, dass mit der Erfindung und deren Weiterbildungen eine Biogasanlagen-Serviceeinrichtung und ein Serviceschacht bereitgestellt werden, welche über eine zuverlässige und normale Funktion hinaus in erhöhtem Maße standfest sind und sich konstruktionsbedingt leicht für den Einsatz an unterschiedlichen Behälter- oder auch Foliendächern anpassen lassen. Dabei lässt sich der Serviceschacht z. B. an vorbestimmte Beabstandungen bzw. Abstände von einer Wand des Fermenterbehälters aus betrachtet und insbesondere auch an unterschiedliche Neigungswinkel des Behälterdaches sehr leicht anpassen.

Vorzugsweise ist das Traggestell insgesamt so ausgebildet, dass es über eine genügende Eigensteifigkeit verfügt, um den zu vorbestimmter Stelle des Behälterdaches ausgerichteten Serviceschacht tragen zu können.

Insbesondere ist die Arbeitsplattform im Wesentlichen horizontal ausgerichtet. Das erleichtert einem Benutzer die Verwendung. Die Arbeitsplattform kann sich direkt an den Tragabschnitt anschließen oder aber beabstandet über einen Zwischenabschnitt gehalten werden. In einer bevorzugten Ausgestaltung kann der Tragabschnitt an der Außenwand angeordnet sein und der Serviceschacht und/oder eine Arbeitsplattform können sich quer dazu auf dem Behälterdach erstrecken. Zwischen dem Tragabschnitt und dem Serviceschacht und/oder der Arbeitsplattform kann ein Zwischenabschnitt vorgesehen sein, der einen gangbaren Weg von der Wand des Behälters zu dem Serviceschacht und/oder der Arbeitsplattform zur Verfügung stellt. Der Tragabschnitt kann senkrecht und die Arbeitsplattform kann horizontal ausgerichtet sein. Der oder ein Zwischenabschnitt kann verschiedenste Winkel annehmen.

Der Tragabschnitt des Traggestells kann vorzugsweise mit Befestigungsmitteln, wie Schrauben oder Dübeln, an der Behälterwand befestigt werden. Der Tragbereich kann an der äußeren Seite der Behälterwand oder auch am oberen Ende der Behälterwand befestigt werden. Möglich ist eine Befestigung auch an der inneren Behälterwand oberhalb des Behälterdaches. Möglich ist es auch, dass der Tragbereich an einem festen Dachsegment oder einem festen Dachelement befestigt wird, welches sich von der Behälterwand aus erstreckt. Möglich ist es auch, Halter in die Behälterwand zu integrieren, an denen das Traggestell befestigt wird.

Der Tragabschnitt der Tragkonsole kann auch an seinem unteren Endbereich in ein Fundament eingelassen sein, das sich an der Außenseite der Behälterwand im Bereich des Bodens des Fermenterbehälters befindet.

Insgesamt wird mit der erfindungsgemäßen Biogasanlagen-Serviceeinrichtung die Möglichkeit eröffnet, auch nachträglich, also bei einer bereits aufgebauten Biogasanlage, einen Serviceschacht an geeigneter Stelle des Daches anbringen zu können, da keine Befestigung des Serviceschachtes im Innenraum an einer Innenseite einer Wand des Fermenterbehälters mehr erforderlich ist. Soweit erfindungsgemäß Biogasanlagen angesprochen sind, ist jedoch zu erwähnen, dass der Einsatz der Serviceeinrichtung mit ihrem Serviceschacht auch bei anderen Behältern möglich ist. Bei besonderer Ausführung des Daches, wie bei glasfaserverstärkten Folien oder anderer Materialien, kann das Dach des Behälters auch begehbar sein.

Vorzugsweise entspricht eine Neigung des Tragabschnitts im Wesentlichen einer Neigung der äußeren Behälterwand. Dabei kann der Tragabschnitt parallel zur Behälterwand angeordnet sein. Möglich ist auch eine etwa senkrechte Anordnung. Der Zwischenabschnitt kann parallel oder etwa parallel zur Dachneigung verlaufen.

Vorzugsweise können die Arbeitsplattform und der Tragabschnitt über wenigstens eine, insbesondere zwei oder mehr Verstärkungsstreben verbunden sein.

Der Tragabschnitt kann als z.B. etwa senkrechter Teilbereich ausgeführt sein. An dem Tragabschnitt können Mittel vorgesehen sein, um den Tragabschnitt in geeignetem Abstand z.B. parallel zur äußeren Seite der Behälterwand auszurichten.

Der Serviceschacht ist insbesondere an ein z.B. als Konsole ausgeführtes Traggestell montiert, das eine zum Serviceschacht hinführende Arbeitsplattform als Teilbereich aufweisen kann. Insbescndere ist die Arbeitsplattform oberhalb des Behälterdaches vorgesehen. Vorzugsweise ist das Behälterdach wenigstens im Bereich des Serviceschachts gegen die Horizontale unter einem Winkel Alpha geneigt.

Der Serviceschacht hat insbesondere wenigstens eine Serviceöffnung, um den Ein- und Ausbau sowie die Wartung von Geräten, wie eines Rührgerätes oder mehrerer Rührgeräte zu erleichtern oder vornehmen zu können. Wenigstens ein Rührgerät ist vorgesehen und insbesondere ist wenigstens ein Rührgerät an der Montagestange vorgesehen oder angebracht.

Der Serviceschacht weist insbesondere wenigstens eine Verstelleinrichtung und insbesondere wenigstens eine Höhenverstelleinrichtung und/oder wenigstens eine Seitenverstelleinrichtung der Rühreinrichtung oder des Rührgerätes auf.

Wenigstens eine Serviceöffnung ist insbesondere als Frontöffnung vorgesehen. Die Serviceöffnung kann eine Frontöffnung und eine zusätzliche Bodenöffnung umfassen. Dabei wird die Frontöffnung durch z.B. eine Frontblende verschlossen und die Bodenöffnung durch eine Bodenblende. Die Bodenöffnung ist vorzugsweise dem Serviceschacht direkt vorgeordnet und ist als verschließbare Öffnung in dem Behälterdach ausgebildet. Die Frontöffnung und die Bodenöffnung bilden zusammen eine große Serviceöffnung, durch die ein Rührgerät aus dem Innenraum in das Freie verbracht werden kann. In der Wartungsposition kann das bzw. ein Rührgerät vollständig innerhalb des Serviceschachtes angeordnet sein oder aber das Rührgerät befindet sich in der wartungsposition nur teilweise oder gar nicht innerhalb des Serviceschachts.

An dem Serviceschacht können wenigstens ein Schauglas und wenigstens eine Anschluss-Gasleitung vorgesehen sein.

Der Serviceschacht ist im Bereich seines Überganges zum Behälterdach vorzugsweise mit wenigstens einer Klemmung versehen, um die Verbindung zum Behälterdach gasdicht und flexibel ausbilden zu können. Es versteht sich dabei von selbst, dass der gesamte Serviceschacht bezüglich seines Innenraumes durch Auswahl geeigneter Materialien, vorzugsweise gasdicht ausgebildet ist.

Der Winkel Alpha der Neigung des Behälterdaches liegt vorzugsweise im Bereich zwischen 10 und 60 Grad.

Der Winkel Alpha der Neigung des Behälterdaches gegen die Horizontale kann beliebig sein und liegt vorzugsweise im Bereich von 10 Grad bis 60 Grad. Eine Serviceeinrichtung bei einem großen Steigungswinkel, z. B. 45 Grad, 60 Grad und mehr wird dabei eigentlich erst durch die von außerhalb des Fermenterbehälters erfolgende Tragkonstruktion des Serviceschachtes ermöglicht.

Es hat sich in diesem Zusammenhang auch herausgestellt, dass das Behälterdach auch als Tragluftdach ausgebildet werden kann, zumal der Gaserzeugungsprozess im Fermenterbehälter stets einen Überdruck gegenüber der Außenatmosphäre auszubilden gestattet.

Als sehr hilfreich hat sich herausgestellt, dass zwischen dem im Bereich der Außenseite der Behälterwand des Fermenterbehälters befindlichen Bodens und der Arbeitsplattform eine Aufstiegsleiter oder Aufstiegstreppe angebracht ist.

Ferner kann eine Montagehilfe dadurch gegeben sein, dass die Konsole oder das Traggestell oberhalb der Arbeitsplattform wenigstens eine Kranbahn aufweist, mittel derer wenigstens ein Kran bzw. ein Schwenkkran, führbar ist. Ein üblicherweise verwendetes Rührgerät kann z. B. eine Masse von 260 kg aufweisen.

Bei der Serviceeinrichtung kann die Montagestange lotrecht in das Behälterinnere und insbesondere in die Fermentermasse hineinragen. Möglich ist aber auch eine geneigte Anordnung der Montage- oder Haltestange.

Die erfindungsgemäße Biogasanlage weist wenigstens einen Fermenterbehälter mit wenigstens einer äußeren Behälterwand und wenigstens einen Fermenterbehälterinnenraum und wenigstens ein den Fermenterbehälterinnenraum abdeckendes Behälterdach auf.

Wenigstens eine Biogasanlage-Serviceeinrichtung mit wenigstens einem Serviceschacht ist vorgesehen. Der Serviceschacht bzw. wenigstens ein Serviceschacht weist wenigstens eine Serviceöffnung auf, um insbesondere den Ein- und Ausbau und/oder die Wartung von Geräten, wie z.B. eines Rührgerätes vornehmen zu können. Die Biogasanlage verfügt vorzugsweise über wenigstens ein Rührgerät in wenigstens einem Fermenterbehälter. Der Serviceschacht ist an dem den Fermenterbehälterinnenraum abdeckenden Behälterdach in einer das Behälterdach abdichtenden Weise angeordnet. Wenigstens eine in den Fermenterbehälterinnenraum und insbesondere in die Fermentermasse hineinragende und von dem Serviceschacht ausgehende Montagestange ist vorgesehen, die von der Behälterwand beabstandet angeordnet ist. Der Serviceschacht ist an wenigstens einem Traggestell befestigt, welches wenigstens einen Tragabschnitt umfasst. An dem Tragabschnitt sind Befestigungsmittel vorgesehen, mit denen der Tragabschnitt ausgerichtet und außerhalb des Fermenterbehälterinnenraums befestigt ist.

Die erfindungsgemäße Biogasanlage kann in bevorzugten Ausgestaltungen mit einem oder mehreren der zuvor beschriebenen Serviceschächte ausgerüstet sein. Insbesondere kann der Serviceschacht wenigstens eine zum Serviceschacht hinführende Arbeitsplattform umfassen.

Weitere vorteilhafte Merkmale und Ausgestaltungen der Erfindung ergeben sich aus dem nachfolgenden Ausführungsbeispiel, welches nun mit Bezug auf die beiliegenden Figuren beschrieben wird.

Darin zeigen:
- Fig 1a: einen erfindungsgemäßen Serviceschacht in einer Ansicht von außerhalb des Fermenterbehälters,
- Fig 1b: eine Schnittansicht durch die Behälterwand mit Sicht auf den Serviceschacht;
- Fig 1c: eine der Teilansicht 1b entsprechende Draufsicht auf einen Bereich des Fermenterbehälters ist;
- Fig 2a: einen weiteren erfindungsgemäßen Serviceschacht in einer Ansicht, teils geschnitten, von außerhalb des Fermenterbehälters;
- Fig 2b: eine Schnittansicht durch die Behälterwand mit Sicht auf den Serviceschacht;
- Fig 2c: eine der Teilansicht 2b entsprechende Draufsicht auf einen Bereich des Fermenterbehälters ist;
- Fig 3: eine Schnittansicht eines Fermenterbehälters einer weiteren Biogasanlage mit einer dritten Ausführungsform eines Serviceschachts; und
- Fig 4: eine Schnittansicht eines Fermenterbehälters einer weiteren Biogasanlage mit einer vierten Ausführungsform eines Serviceschachts.

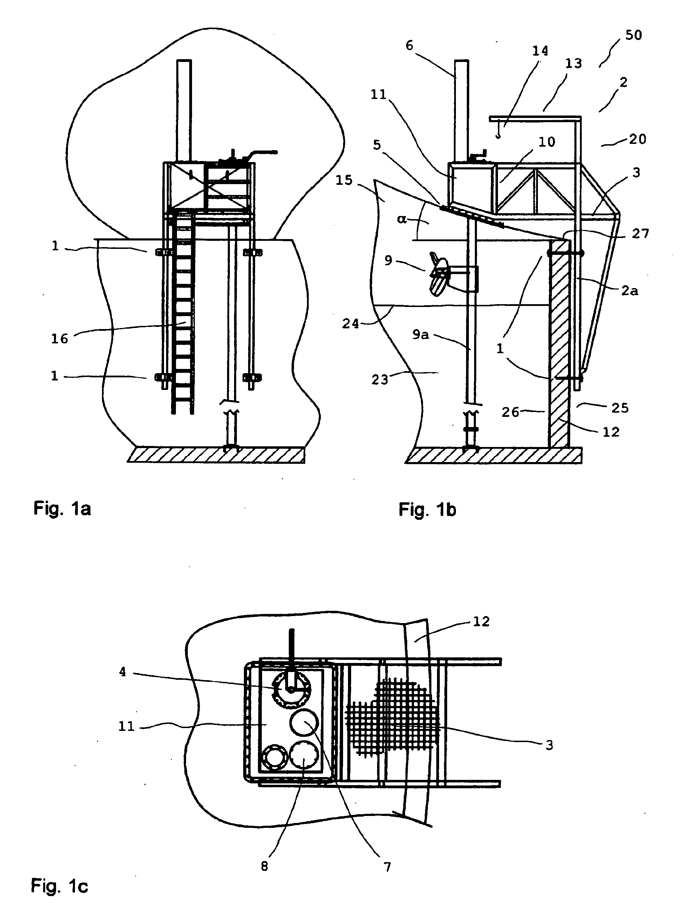

In den Figuren 1a bis 1c ist ein Teil einer ersten erfindungsgemäßen Biogasanlage 50 mit einer Biogasanlagen-Serviceeinrichtung 20 dargestellt, die einen Serviceschacht 11 aufweist.

Die Biogasanlage 50 weist einen Fermenterbehälter 21 mit einem Innenraum 22 und einer Behälterwand 12 auf. Der Innenraum 22 wird nach oben von einem Behälterdach 15 begrenzt, welches dichtend an der Behälterwand 12 angebracht ist.

Der Serviceschacht 11 ist an dem hier als Foliendach ausgeführtem Behälterdach 15 vorgesehen.

Der Serviceschacht der Serviceeinrichtung 20 weist hier eine Serviceöffnung 10 auf, die einen Zugriff auf oder einen Zugang in den Innenraum 22 des Fermenterbehälters 21 erlaubt.

An dem Serviceschacht ist eine Montagestange 9a vorgesehen, die sich von dem Serviceschacht aus nach innen (und gegebenenfalls auch nach außen) erstreckt. Die Montagestange 9a endet am unteren Ende vorzugsweise am Boden 29 des Fermenterbehälters 21 und ist dort gegebenenfalls drehbar befestigt. Die Montagestange 9a kann senkrecht ausgerichtet oder auch geneigt ausgerichtet sein.

An der Montagestange 9a ist wenigstens ein Rührgerät 9 höhenverstellbar und auch seitlich verschwenkbar aufgenommen. Das Rührgerät 9 kann elektrisch, hydraulisch oder sonst wie angetrieben werden. Das Rührgerät 9 dient dazu, die Fermentermasse umzurühren und zu homogenisieren. Weiterhin soll es Ablagerungen und Sedimentationen verhindern. Aufgrund der abrasiven und aggressiven Umgebung in der Fermentermasse muss das Rührgerät 9 von Zeit zu Zeit gewartet werden. Beispielsweise müssen die Rührflügel bei Bedarf ausgetauscht werden. Bei Defekten muss das Rührgerät 9 auch repariert oder ausgetauscht werden.

Durch die Serviceöffnung 10 ist das Rührgerät 9 zugänglich. Mittels der Höhenverstelleinrichtung 30 der Verstellungseinrichtung 4 kann das Rührgerät 9 nach oben verfahren werden. Das Rührgerät 9 wird dabei oberhalb der Oberfläche 24 des Flüssigkeitsspiegels der Fermentermasse 23 und insbesondere bis nach oben in den Freiraum in dem Serviceschacht 11 oberhalb des Behälterdaches 15 oder ganz ins Freie verfahren, damit eine Bedienperson das Rührgerät einfach zugänglich warten oder auch austauschen kann. Das Foliendach 15 muss dazu nicht geöffnet werden. Zur Wartung kann das Rührgerät 9 auch aus dem Inneren des Serviceschachts herausgeschwenkt werden.

Der Serviceschacht 11 ist an einem Traggestell 2 befestigt, das als Konsole 2 ausgebildet sein kann, aber nicht muss. Das Traggestell 2 umfasst hier einen Tragabschnitt 2a und eine zum Serviceschacht 11 hinführende Arbeizsplattform. An dem Tragabschnitt 2a sind Befestigungsmittel 1 vorgesehen, mit denen der Tragabschnitt 2a ausgerichtet und außerhalb des Fermenterbehälterinnenraums 2 befestigt wird.

Dabei ist der Tragabschnitt 2a außerhalb des Fermenterbehälterinnenraums 22 befestigt.

Der Tragabschnitt 2a kann insbesondere an der Außenseite 25 der Behälterwand 12 befestigt werden, wie es Fig. 1 zeigt. Möglich ist es auch, den Tragabschnitt 2a an dem oberen Ende bzw. der Oberseite 27 der Behälterwand 12 und/oder oberhalb des Behälterdaches 15 an der Innenseite 26 der Behälterwand 12 zu befestigen, wenn sich die Behälterwand noch über den Ansatz des Behälterdaches aus noch weiter nach oben erstreckt.

Der Serviceschacht 11 wird im Ausführungsbeispiel nach Fig. 1 an der Außenwand des Fermenterbehälters 21 gehaltert. Dazu dienen im Ausführungsbeispiel nach Fig. 1 insbesondere Schrauben und Dübel als Befestigungsmittel 1. In die in die Außenseite der Behälterwand 12 eingeführten Dübel werden die Schrauben eingeschraubt, um die Konsole 2 über den Tragabschnitt 2a an der Außenseite des Behälters zu befestigen. Möglich ist es auch, Befestigungsmittel in die Behälterwand einzugießen, wenn der Behälter z.B. aus Beton oder Kunststoff besteht oder aber Befestigungsmittel anzuschweißen, wenn der Behälter teilweise aus Metall und insbesondere Stahl besteht.

Der Serviceschacht 11 ist oberhalb des Daches des Fermenterbehälters 21 angeordnet. Der Serviceschacht 11 ist hier an eine Konsole 2 als Traggestell 2 montiert.

Die Konsole 2 hat hier im Ausführungsbeispiel einen im Wesentlichen rechtwinkligen Aufbau und ist insbesondere ausreichend eigensteif zusammengebaut, z. B. mit Streben, die Dreiecke oder dergleichen bilden und so die beispielsweise gewählte Rahmenkonstruktion der Konsole stabil machen.

Der hier waagrechte Teil der Konsole 2 gibt eine Arbeitsplattform 3 ab, die zu dem Serviceschacht 11 führt, der an die Konsole 2 montiert ist und diese trägt.

Ein Tragabschnitt 2a der Konsole 2, der hier im Ausführungsbeispiel als senkrechter Teilbereich ausgeführt ist, kann direkt mit Schrauben oder Dübeln 1 an der Außenseite einer Behälterwand 12 des Fermenterbehälters befestigt werden, wie dies in Fig. 1b dargestellt ist.

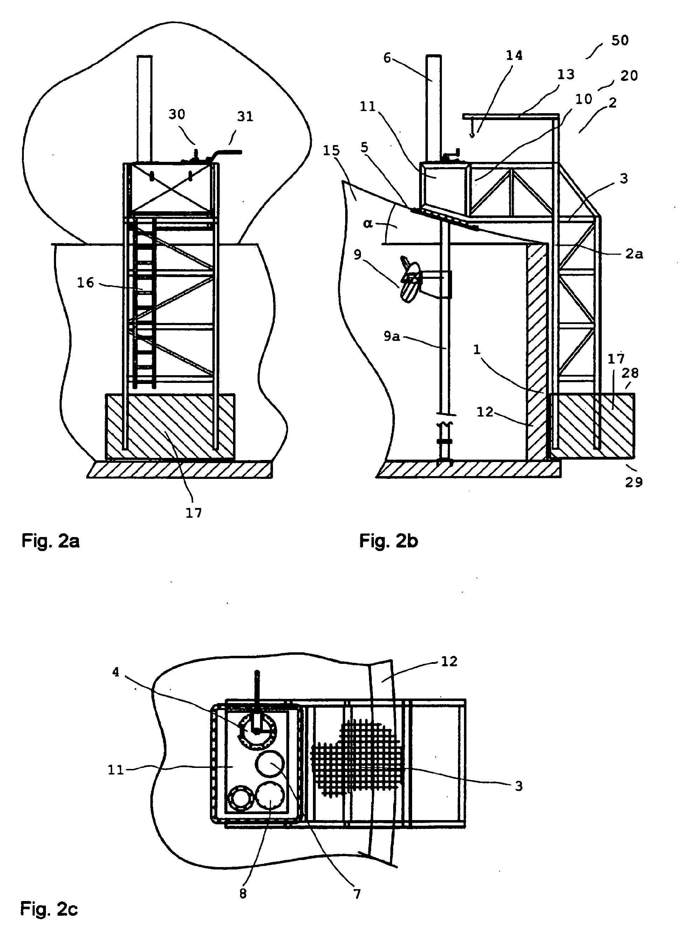

In dem Ausführungsbeispiel nach den Figuren 2a bis 2c ist ein weiterer erfindungsgemäßer Serviceschacht 11 dargestellt, der oberhalb des Behälterdaches des Fermenterbehälters angeordnet ist.

Hier ist der Tragabschnitt 2a an seinem unteren Endbereich 28 in ein Fundament 17 eingelassen ist, das sich an der Außenseite 25 der Behälterwand 12 im Bereich des Bodens 29 des Fermenterbehälters befindet.

Bei diesem Ausführungsbeispiel ist ein Fundament 17 vorgesehen, das sich beispielsweise an der Außenseite 25 der Behälterwand 12 im unteren Bereich der Außenwand 12 des Fermenterbehälters 21 oder im Bereich des Bodens 29 des Fermenterbehälters 21 befindet, wie dies in Fig. 2a und Fig. 2b dargestellt ist. Hier ist der Tragabschnitt 2a der Konsole 2 an dem Fundament 17 befestigt.

Es sind jedoch auch andere geeignete Platzierungen des Fundamentes möglich. Wichtig ist nur, dass bei dieser Ausgestaltung der untere Endbereich 28 des Tragabschnitts 2a der Konsole 2 fest in dem Fundament 17 verankert werden kann, z. B. durch Einbetonieren, sodass der eigensteife Aufbau der Konsole 2 den Serviceschacht 11 auch stabil zu tragen vermag. Dabei kann es auch wichtig sein, dass die Masse des Fundamentes 17 ausreichend bestimmt ist. Das Material der Konsole, insbesondere das Material des senkrechten Teilbereichs 2a, soll dann auch zum Einbetonieren geeignet sein oder von einem entsprechend geeigneten Material umgeben sein.

Der Serviceschacht 11 selbst ist so ausgestaltet, dass er eine Serviceöffnung hat, die hier als Frontöffnung 10 ausgeführt ist. Mit der Frontöffnung 10 kann der Ein- und Ausbau eines Gerätes vorgenommen werden. Beispielsweise kann ein Rührgerät 9 oder einer Pumpe ein- oder ausgebaut werden.

Insbesondere ermöglicht die Frontöffnung 10 die Wartung von Geräten und insbesondere des Rührgeräts 9. Es ist vorgesehen, dass der Serviceschacht 11 eine Höhen- und Seitenverstellung 4 mit einer Höhenverstellung 30 und einer Seitenverstellung 31 des Rührgerätes aufweist. Die Rührflügel können so auf einfache Weise ausgetauscht werden.

Dabei ist es nicht nötig, den Füllstand des Fermenterbehälters 21 abzusenken. Auch das Behälterdach muss hier nicht ganz oder teilweise entfernt werden.

Wenigstens eine Anschluss- bzw. Gasleitung 8 dient zum Abführen des erzeugten Biogases.

Das elektrisch oder insbesondere hydraulisch betriebene Rührgerät 9 ist bevorzugt an einer vom Serviceschacht 11 in die Fermentermasse hineinragenden Montagestange 9a angebracht. Die Montagestange 9a kann lotrecht angeordnet sein.

Von dem Serviceschacht 11 ausgehend kann das Warten der eingebauten Geräte, wie des Rührgerätes 9 vorgenommen werden. Darüber hinaus können auch Kontrollen der eingebauten Geräte vorgenommen werden. Dies kann auch direkt von der Arbeitsplattform 3 aus erfolgen. Bei einer Wartung kann sich das Rührgerät 9 teilweise oder auch vollständig außerhalb des Innenraumes des Serviceschachtes befinden.

Ferner kann das Gärsubstrat durch ein Schauglas beobachtet werden, das z. B. auch im Bereich der Arbeitsplattform 3 untergebracht werden kann.

Der Serviceschacht 11 ist gasdicht und flexibel mit der Folie des Behälterdaches 15 verbunden. Dafür ist eine spezielle Klemmung 5 zwischen der schrägen Unterseite des Serviceschachtes 11 und der Folie des Behälterdaches vorgesehen. Die Klemmung kann an die Dachneigung angepasst sein.

Der Serviceschacht 11 selbst soll im geschlossenen Zustand, wie das auch im Stand der Technik üblich ist, gasdicht sein. Es ist deshalb an der oberen Seite (Dachseite) dieses Serviceschachtes 11 eine Überdruck- bzw. auch Unterdrucksicherung 6 vorgesehen. Zwischen der Arbeitsplattform 3 und dem Eingangsbereich des Serviceschachtes 11 kann die bereits erwähnte Frontöffnung 10, wie eine Tür, vorgesehen sein, die eine ausreichende Abdichtung bewirkt bzw. aufweist. Zusätzlich kann in dem Boden der Arbeitsplattform eine durch eine Bodenblende normalerweise gasdicht verschlossene Bodenöffnung vorgesehen sein, sodass im geöffneten Zustand beide Öffnungen gemeinsam die Serviceöffnung ergeben, durch die ein Rührgerät 9 aus dem Inneraum 22 nach außen oberhalb des Behälterdaches verbracht werden kann, um eine Wartung, Reparatur oder einen Austausch durchzuführen.

Der Winkel Alpha der Dachneigung kann beliebig sein. Die Ausführungsformen der Erfindung sind besonders bevorzugt auf alle und auch auf große bzw. größere Dachneigungen ausgerichtet.

Der Behälter kann aus verschiedensten Materialien bestehen. Möglich und bevorzugt ist insbesondere der Einsatz von Fermenterbehältern, die teilweise oder vollständig aus Stein, Beton, Metallen oder Metalllegierungen und insbesondere Stahl oder aus sonstigen Natur- oder Kunststoffen bestehen. Besonders bevorzugt bestehen Fermenterbehälter aus Beton oder Stahl. Das Dach kann ebenfalls aus verschiedensten Materialien bestehen. Besonders bevorzugt ist Beton, Folie oder insbesondere auch Stahl.

Bei größeren Dachneigungen ist eine Befestigung und Abstützung des Serviceschachtes von der Innenseite des Fermenterbehälters relativ aufwändig. Erfindungsgemäße Ausführungsformen ermöglichen auch Steigungswinkel Alpha von 30 Grad und mehr oder von 45 Grad und mehr. Möglich sind jedoch auch kleinere Steigungswinkel zwischen z. B. 5 Grad und 30 Grad. Grundsätzlich ist auch der Einsatz an einem Flachdach möglich. Neben einer Ausbildung als Foliendach kann das Behälterdach wenigstens teilweise oder vollständig auch aus einem festen Material, wie Stein, Beton oder Stahl bestehen.

Als Behälterdach 15 kann auch ein Tragluftdach vorgesehen sein. Dafür eignet sich der nach dem Ausführungsbeispiel ausgebildete Serviceschacht 11 besonders gut, da der Serviceschacht 11 grundsätzlich nicht von innen an der Wand befestigt wird, sondern oben an der Behälterwand oder aber außenseitig. Das praktische Arbeiten mit dem Serviceschacht 11 wird sehr erleichtert, wenn im Bereich der Außenseite der Behälterwand 12 hinführend zur Arbeitsplattform 3 eine Aufstiegsleiter 16 angebracht ist. Alternativ dazu kann eine Treppe vorgesehen sein.

In die Arbeitsplattform ist hier zur Erhöhung der Arbeitssicherheit ein rutschfester Gitterrost integriert. Ferner werden die eigentlichen Montagearbeiten erheblich erleichtert und unerstützt, wenn oberhalb der Arbeitsplattform 3 eine Kranbahn 13 angebracht ist, mit Hilfe derer ein Kran 14 führbar ist.

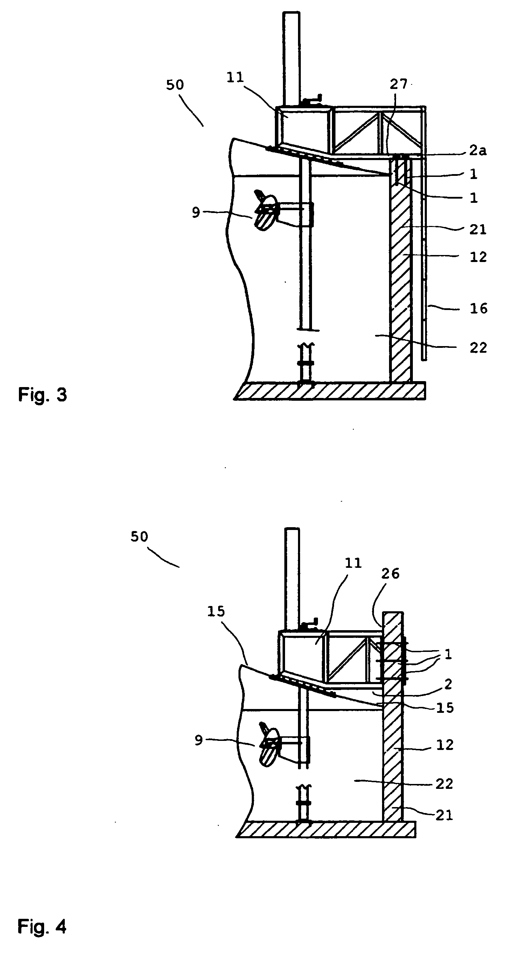

In Fig. 3 ist ein Fermenterbehälter 21 einer Biogasanlage 50 in einem Teilschnitt dargestellt, wobei der Serviceschacht 11 der Serviceeinrichtung 20 über das Traggestell 2 an dem oberen Ende der Behälterwand 12 angebracht ist. Dazu ist der Tragabschnitt 2a insbesondere direkz über Befestigungsmittel 1 mit der Oberseite 27 der Behälterwand verschraubt. Eine Leiter 16 oder dergleichen kann vorgesehen sein, um den Aufstieg zu erleichtern. Die weiteren Komponenten entsprechen denen der vorangegangenen Ausführungsbeispiele.

Fig. 4 zeigt einen weiteren Fermenterbehälter 21 einer weiteren Biogasanlage 50 in einem Teilschnitt. Dabei ist der Serviceschacht 11 der Serviceeinrichtung 20 über das Traggestell 2 an der Innenseite 26 der Behälterwand 12 angebracht. Der Befestigungspunkt mit der Behälterwand 12 ist oberhalb des Behälterdaches 15, sodass der Tragabschnitt außerhalb des Innenraums 22 des Fermenterbehälters 21 festgelegt und ausgerichtet ist. Hier erstreckt sich die Behälterwand 12 des Fermenterbehälters 21 über den Ansatz des Daches hinaus nach oben. Dadurch wird eine Befestigung auf der Innenseite 26 ermöglicht, ohne dass der Tragabschnitt ständig der Fermentermasse ausgesetzt ist. Auch eine Störung der Strömung erfolgt nicht.

Auch hier kann eine Leiter 16 oder dergleichen vorgesehen sein, um den Aufstieg zu erleichtern. Die weiteren Komponenten entsprechen wieder denen der vorangegangenen Ausführungsbeispiele.

Mit der Erfindung wird insgesamt eine kostengünstige und einfach zu montierende Serviceeinrichtung zur Verfügung gestellt, welche bei allen Arten von Dächern große Vorteile bringt.

Die beiliegenden Figuren zeigen sie Befestigung an der Behälterwand oder an dem Fundament des Behälters. Möglich ist auch ein separates Fundament, welches unabhängig von dem Behälter ist.

Der Serviceschacht ist für jedes Behälterdach 15 und jede Dachneigung geeignet und kann für fast alle Dächer 15 nachträglich montiert werden.

Weitere Ausgestaltungen und Merkmale können realisiert sein, wie es in der europäischen Patentschrift EP 1 717 305 B1 beschrieben ist, auf die insofern Bezug genommen wird.

Die Erfindung kann an Fermenterbehältern von Biogasanlagen eingesetzt werden. Möglich ist auch der Einsatz an anderen Behältern. Die obere Abdeckung kann trittfest und somit begehbar ausgeführt sein, kann aber auch als dünne Folie oder dergleichen ausgeführt sein.

Die Arbeitsplattform 3 dient zum Warten und Kontrollieren der eingebauten Geräte 9 und zur Beobachtung des Gärsubstrats durch das Schauglas 7.

### Bezugszeichenliste:

- 1: Befestigungsmittel, Dübel
- 2: Konsole
- 2a: Tragabschnitt
- 3: Arbeitsplattform
- 4: Höhen- und Seitenverstellung des Rührgerätes
- 5: Klemmung
- 6: Über- und Unterdrucksicherung
- 7: Schauglas
- 8: Anschluss-Gasleitung
- 9: Rührgerät
- 9a: Montagestange
- 10: Frontöffnung
- 11: Serviceschacht
- 12: Behälterwand
- 13: Kranbahn
- 14: Kran
- 15: Behälterdach, Foliendach
- 16: Aufstiegsleiter
- 17: Fundament
- 20: Serviceeinrichtung
- 21: Fermenterbehälter
- 22: Innenraum
- 23: Fermentermasse
- 24: Oberfläche
- 25: Außenseite
- 26: Innenseite
- 27: Oberseite
- 28: unterer Endbereich
- 29: Boden des Fermenterbehälters
- 30: Höhenverstelleinrichtung
- 31: Seitenverstelleinrichtung
- 50: Biogasanlage
- α: Alpha, Dachneigungswinkel

## Patentansprüche

1. Biogasanlagen-Serviceeinrichtung (20), mit wenigstens einem Serviceschacht (11) für Biogasanlagen (50), welche wenigstens einen Fermenterbehälter (21) mit wenigstens einer Behälterwand (12) und wenigstens einen Fermenterbehälterinnenraum (22) und wenigstens ein den Fermenterbehälterinnenraum (22) abdeckendes Behälterdach (15) aufweisen,
wobei der Serviceschacht (11) wenigstens eine Serviceöffnung (10) aufweist, um insbesondere den Ein- und Ausbau und/oder die Wartung von Geräten, wie z.B. eines Rührgerätes (9) zu erleichtern,
wobei der Serviceschacht (11) an einem den Fermenterbehälterinnenraum (22) abdeckenden Behälterdach (15) in einer das Behälterdach (15) abdichtenden Weise anordnenbar ist, und wobei wenigstens eine in den Fermenterbehälterinnenraum (22) und insbesondere in die Fermentermasse (23) hineinragende und von dem Serviceschacht (11) ausgehende Montagestange (9a) vorgesehen ist, die von der Behälterwand (12) beabstandet anordnenbar ist,
**dadurch gekennzeichnet,**
**dass** der Serviceschacht (11) an einem Traggestell (2) befestigt ist, welches wenigstens einen Tragabschnitt (2a) umfasst, wobei an dem Tragabschnitt (2a) Befestigungsmittel (1) vorgesehen sind, mit denen der Tragabschnitt (2a) ausrichtbar und außerhalb des Fermenterbehälterinnenraums (21) befestigbar ist.

2. Biogasanlagen-Serviceeinrichtung (20) nach Anspruch 1, wobei der Tragabschnitt (2a) außerhalb des Fermenterbehälterinnenraums (22) an der Behälterwand (12) befestigt ist.

3. Biogasanlagen-Serviceeinrichtung (20) nach Anspruch 1 oder 2, wobei der Tragabschnitt (2a) an der Außenseite (25) der Behälterwand (12) und/oder an dem oberen Ende (27) der Behälterwand (12) und/oder oberhalb des Behälterdaches (15) an der Innenseite (26) der Behälterwand (12) befestigt ist.

4. Biogasanlagen-Serviceeinrichtung (20) nach mindestens einem der vorhergehenden Ansprüche, wobei der Tragabschnitt (2a) an seinem unteren Endbereich (28) in ein Fundament (17) eingelassen ist, das sich an der Außenseite (25) der Behälterwand (12) im Bereich des Bodens (29) des Fermenterbehälters befindet.

5. Biogasanlagen-Serviceeinrichtung (20) nach mindestens einem der vorhergehenden Ansprüche, wobei das Traggestell (2) weiterhin wenigstens eine Arbeitsplattform (3) umfasst, welche insbesondere dem Serviceschacht (11) vorgelagert ist und wobei insbesondere das Traggestell (2) insgesamt so ausgebildet ist, dass die Arbeitsplattform (3) und der Tragabschnitt (2a) über wenigstens eine Verstärkungsstrebe verbunden sind, sodass das Traggestell (2) über eine genügende Eigensteifigkeit verfügt, um den zu einer vorbestimmten Stelle des Behälterdaches (15) ausgerichteten Serviceschacht (11) tragen zu können.

6. Biogasanlagen-Serviceeinrichtung (20) nach mindestens einem der vorhergehenden Ansprüche, wobei die Arbeitsplattform im Wesentlichen horizontal ausgerichtet ist und/oder wobei eine Neigung des Tragabschnitts im Wesentlichen einer Neigung der äußeren Behälterwand (12) entspricht und wobei der Tragabschnitt vorzugsweise etwa senkrecht angeordnet ist.

7. Biogasanlagen-Serviceeinrichtung (20) nach mindestens einem der vorhergehenden Ansprüche, wobei das Behälterdach (15) wenigstens teilweise als Foliendach (15) ausgebildet ist oder ein solches umfasst, und wobei das Behälterdach (15) insbesondere wenigstens teilweise als ein Tragluftdach ausgebildet sein kann oder ein solches umfasst.

8. Biogasanlagen-Serviceeinrichtung (20) nach mindestens einem der vorhergehenden Ansprüche, wobei das Behälterdach (15) im Bereich des Serviceschachtes gegen die Horizontale unter einem Winkel Alpha geneigt ist.

9. Biogasanlagen-Serviceeinrichtung (20) nach mindestens einem der vorhergehenden Ansprüche, wobei wenigstens ein Rührgerät an der Montagestange (9a) vorgesehen und vorzugsweise angebracht ist und/oder wobei der Serviceschacht (11) wenigstens eine Verstelleinrichtung (4) und insbesondere wenigstens eine Höhenverstelleinrichtung (30) und/oder eine Seitenverstelleinrichtung (31) des Rührgerätes (9) aufweist.

10. Biogasanlagen-Serviceeinrichtung (20) nach mindestens einem der vorhergehenden Ansprüche, wobei der Serviceschacht (11) wenigstens ein Schauglas (7) und wenigstens eine Anschluss-Gasleitung (8) aufweist.

11. Biogasanlagen-Serviceeinrichtung (20) nach mindestens einem der vorhergehenden Ansprüche, wobei der Serviceschacht (11) mit der Folie des Foliendaches (15) mittels einer Klemmung (5) gasdicht und flexibel verbunden ist.

12. Biogasanlagen-Serviceeinrichtung (20) nach mindestens einem der vorhergehenden Ansprüche, wobei der Winkel Alpha der Neigung des Behälterdaches im Bereich zwischen 10 und 60 Grad liegt.

13. Biogasanlagen-Serviceeinrichtung (20) nach mindestens einem der vorhergehenden Ansprüche, wobei an der Außenseite (25) der Behälterwand (12) des Fermenterbehälters wenigstens eine Aufstiegseinrichtung wie insbesondere eine Aufstiegsleiter (16) oder eine Aufstiegstreppe angebracht ist.

14. Biogasanlagen-Serviceeinrichtung (20) nach mindestens einem der vorhergehenden Ansprüche, wobei das Traggestell (2) oberhalb der Serviceöffnung wenigstens eine Kranbahn (13) aufweist, mittels derer wenigstens ein Kran (14) führbar ist.

15. Biogasanlage (50) mit wenigstens einem Fermenterbehälter (21) mit wenigstens einer Behälterwand (12) und wenigstens einen Fermenterbehälterinnenraum (22) und wenigstens einem den Fermenterbehälterinnenraum (22) abdeckenden Behälterdach (15),
wobei wenigstens eine Biogasanlage-Serviceeinrichtung (20) mit wenigstens einem Serviceschacht (11) vorgesehen ist, wobei der Serviceschacht (11) wenigstens eine Serviceöffnung (10) aufweist, um insbesondere den Ein- und Ausbau und/oder die Wartung von Geräten, wie z.B. eines Rührgerätes (9) zu erleichtern,
wobei der Serviceschacht (11) an einem den Fermenterbehälterinnenraum (22) abdeckenden Behälterdach (15) in einer das Behälterdach (15) abdichtenden Weise angeordnet ist, und wobei wenigstens eine in den Fermenterbehälterinnenraum (22) und insbesondere in die Fermentermasse (23) hineinragende und von dem Serviceschacht (11) ausgehende Montagestange (9a) vorgesehen ist, die von der Behälterwand (12) beabstandet angeordnet ist,
**dadurch gekennzeichnet,**
**dass** der Serviceschacht (11) an einem Traggestell (2) befestigt ist, welches wenigstens einen Tragabschnitt (2a) umfasst, wobei an dem Tragabschnitt (2a) Befestigungsmittel (1) vorgesehen sind, mit denen der Tragabschnitt (2a) ausrichtbar und außerhalb des Fermenterbehälterinnenraums (21) befestigbar ist.

## Claims

1. Biogas plant servicing device (20) having at least one servicing well (11) for biogas plants (50) comprising at least one fermenter tank (21) having at least one tank wall (12) and at least one fermenter tank interior (22) and at least one tank cover (15) covering the fermenter tank interior (22), wherein the servicing well (11) comprises at least one servicing opening (10) to facilitate in particular installation and removal and/or maintenance of devices such as an agitator (9), wherein the servicing well (11) can be disposed at a tank cover (15) covering the fermenter tank interior (22) so as to seal the tank cover (15), and wherein at least one mounting rod (9a) is provided which projects into the fermenter tank interior (22) and in particular into the fermenter mass (23) and starts from the servicing well (11) and which can be disposed spaced from the tank wall (12), **characterized in that** the servicing well (11) is fastened to a supporting stand (2) comprising at least one supporting section (2a) wherein the supporting section (2a) is provided with fasteners (1) with which the supporting section (2a) can be oriented and fastened outside the fermenter tank interior (21).

2. The biogas plant servicing device (20) according to claim 1 wherein the supporting section (2a) is fastened to the tank wall (12) outside the fermenter tank interior (22).

3. The biogas plant servicing device (20) according to claim 1 or 2 wherein the supporting section (2a) is fastened to the outside surface (25) of the tank wall (12) and/or to the top end (27) of the tank wall (12) and/or above the tank cover (15) to the inside surface (26) of the tank wall (12).

4. The biogas plant servicing device (20) according to at least one of the preceding claims wherein the supporting section (2a) at its lower end region (28) is embedded in a foundation (17) located on the outside surface (25) of the tank wall (12) in the region of the bottom (29) of the fermenter tank.

5. The biogas plant servicing device (20) according to at least one of the preceding claims wherein the supporting stand (2) furthermore comprises at least one working platform (3) which is in particular disposed immediately preceding the servicing well (11) and wherein in particular the supporting stand (2) is on the whole configured such that the working platform (3) and the supporting section (2a) are connected through at least one reinforcing strut such that the supporting stand (2) has sufficient inherent stiffness for carrying the servicing well (11) that is oriented at a predetermined spot of the tank cover (15).

6. The biogas plant servicing device (20) according to at least one of the preceding claims wherein the working platform is substantially oriented horizontally and/or wherein an inclination of the supporting section substantially corresponds to an inclination of the outer tank wall (12) and wherein the supporting section is preferably arranged approximately vertical.

7. The biogas plant servicing device (20) according to at least one of the preceding claims wherein the tank cover (15) is at least partially configured as or comprises top sheeting (15) and wherein the tank cover (15) may in particular at least partially be configured as, or comprise, an air-supported roof.

8. The biogas plant servicing device (20) according to at least one of the preceding claims wherein the tank cover (15) is inclined at an angle alpha relative to the horizontal in the region of the servicing well.

9. The biogas plant servicing device (20) according to at least one of the preceding claims wherein at least one agitator is provided at and preferably attached to the mounting rod (9a) and/or wherein the servicing well (11) comprises at least one adjusting device (4) and in particular at least one height adjusting device (30) and/or a lateral adjusting device (31) of the agitator (9).

10. The biogas plant servicing device (20) according to at least one of the preceding claims wherein the servicing well (11) comprises at least one viewing window (7) and at least one connecting gas line (8).

11. The biogas plant servicing device (20) according to at least one of the preceding claims wherein the servicing well (11) is connected with the sheet of the top sheeting (15) by means of clamping (5) to be gas-tight and flexible.

12. The biogas plant servicing device (20) according to at least one of the preceding claims wherein the angle alpha of the tank cover inclination lies in the range between 10 and 60 degrees.

13. The biogas plant servicing device (20) according to at least one of the preceding claims wherein at least one ascension device such as in particular an ascension ladder (16) or ascension stairs are attached to the outside surface (25) of the tank wall (12) of the fermenter tank

14. The biogas plant servicing device (20) according to at least one of the preceding claims wherein the supporting stand (2) comprises at least one crane track (13) above the servicing opening by means of which at least one crane (14) can be guided.

15. A biogas plant (50) having at least one fermenter tank (21) having at least one tank wall (12) and at least one fermenter tank interior (22) and at least one tank cover (15) covering the fermenter tank interior (22), wherein at least one biogas plant servicing device having (20) at least one servicing well (11) is provided, wherein the servicing well (11) comprises at least one servicing opening (10) to facilitate in particular installation and removal and/or maintenance of devices such as an agitator (9), wherein the servicing well (11) is disposed at the tank cover (15) covering the fermenter tank interior (22) so as to seal the tank cover (15), and wherein at least one mounting rod (9a) is provided which projects into the fermenter tank interior (22) and in particular into the fermenter mass (23) and starts from the servicing well (11) and which is disposed spaced from the tank wall (12), **characterized in that** the servicing well (11) is fastened to a supporting stand (2) comprising at least one supporting section (2a) wherein the supporting section (2a) is provided with fasteners (1) with which the supporting section (2a) can be oriented and fastened outside the fermenter tank interior (21).

## Revendications

1. Dispositif de service pour installations de méthanisation (20), doté au moins d'une trémie pour l'entretien (11) d'installations de méthanisation (50), lesquelles présentent au moins une cuve de digestion (21) munie au moins d'une paroi de cuve (12), au moins d'un espace intérieur de digesteur (22) et au moins d'un toit de cuve (15) recouvrant l'espace intérieur du digesteur (22), la trémie d'entretien (11) présentant au moins un orifice (10), notamment pour faciliter le montage et le démontage et/ou l'entretien d'appareils comme par exemple d'un agitateur (9),
la trémie d'entretien (11) pouvant être agencée sur un toit de cuve (15) recouvrant l'intérieur du digesteur (22) de manière à rendre hermétique le toit de cuve (15), et au moins une tige de montage (9a) étant prévue pénétrant à l'intérieur du digesteur (22) et notamment dans la masse de fermentation (23) et venant de la trémie (11), cette tige pouvant être agencée à une certaine distance de la paroi de cuve (12),
**caractérisé en ce**
**que** la trémie d'entretien (11) est fixée à un cadre de support (2) qui comprend au moins une zone de support (2a), des moyens de fixation (1) étant prévus au niveau de la zone de support (2a), avec lesquels la zone de support (2a) peut être orientée et fixée en dehors de l'espace intérieur du digesteur (21).

2. Dispositif de service pour installations de méthanisation (20) selon la revendication 1, la zone de support (2a) étant fixée à la paroi de la cuve (12) en dehors de l'espace intérieur du digesteur (22).

3. Dispositif de service pour installations de methanisation (20) selon l'une quelconque des revendications 1 ou 2, la zone de support (2a) étant fixée à la face externe (25) de la paroi de cuve (12) et/ou à l'extrémité supérieure (27) de la paroi de cuve (12) et/ou au-dessus du toit de cuve (15) à la face interne(26) de la paroi de cuve (12).

4. Dispositif de service pour installations de méthanisation (20) selon au moins l'une quelconque des revendications précédentes, la zone de support (2a) étant intégrée au niveau de son extrémité inférieure (28) dans des fondations (17) qui se trouvent sur la face externe (25) de la paroi de cuve (12) au niveau du fond (29) du digesteur.

5. Dispositif de service pour installations de méthanisation (20) selon au moins l'une quelconque des revendications précédentes, le cadre de support (2) comprenant encore au moins une plateforme de travail (3) qui est disposée notamment en amont de la trémie d'entretien (11) et le cadre de support (2) notamment étant formé en général de telle sorte que la plateforme de travail (3) et la zone de support (2a) soient reliées par au moins un tirant de renfort, de telle sorte que le cadre de support (2) dispose d'une rigidité propre suffisante pour pouvoir supporter la trémie d'entretien (11) orientée par rapport à un point déterminé du toit de cuve (15).

6. Dispositif de service pour installations de méthanisation (20) selon au moins l'une quelconque des revendications précédentes, la plateforme de travail étant sensiblement orientée à l'horizontale et/ou une inclinaison de la zone de support correspondant sensiblement à une inclinaison de la paroi externe de cuve (12) et la zone de support étant agencée de préférence à peu près à la verticale.

7. Dispositif de service pour installations de méthanisation (20) selon au moins l'une quelconque des revendications précédentes, le toit de cuve (15) étant conçu au moins partiellement en tant que membrane souple (15) ou comprenant une telle membrane, et le toit de cuve (15) pouvant être conçu notamment au moins partiellement comme un toit à double membrane ou en comprenant un tel.

8. Dispositif de service pour installations de méthanisation (20) selon au moins l'une quelconque des revendications précédentes, le toit de cuve (15) étant incliné d'un angle alpha en-dessous de l'horizontale au niveau de la trémie d'entretien.

9. Dispositif de service pour installations de méthanisation (20) selon au moins l'une quelconque des revendications précédentes, au moins un agitateur étant prévu et monté de préférence à la tige de montage (9a), et/ou la trémie d'entretien (11) présentant au moins un dispositif de réglage (4) et notamment au moins un dispositif de réglage de hauteur (30) et/ou un dispositif de réglage latéral (31) de l'agitateur (9).

10. Dispositif de service pour installations de méthanisation (20) selon au moins l'une quelconque des revendications précédentes, la trémie d'entretien (11) présentant au moins un regard (7) et au moins une conduite de gaz de raccordement (8).

11. Dispositif de service pour installations de méthanisation (20) selon au moins l'une quelconque des revendications précédentes, la trémie d'entretien (11) étant reliée à la membrane du toit (15) par serrage (5) de façon souple et hermétique au gaz.

12. Dispositif de service pour installations de méthanisation (20) selon au moins l'une quelconque des revendications précédentes, l'angle alpha de l'inclinaison du toit se situant entre 10 et 60 degrés.

13. Dispositif de service pour installations de méthanisation (20) selon au moins l'une quelconque des revendications précédentes, au moins un dispositif d'ascension tel qu'une échelle (16) ou un escalier étant monté sur la face externe (25) de la paroi de cuve (12) du digesteur.

14. Dispositif de service pour installations de méthanisation (20) selon au moins l'une quelconque des revendications précédentes, le cadre de support (2) présentant au-dessus de l'orifice de regard au moins un chemin de roulement (13) au moyen duquel au moins une grue (14) peut être guidée.

15. Installations de méthanisation (50) dotées au moins d'une cuve de digestion (21) munie au moins d'une paroi de cuve (12) et au moins d'un espace intérieur de digesteur (22) et au moins d'un toit de cuve (15) recouvrant l'intérieur du digesteur (22),
au moins un dispositif de service pour installations de méthanisation (20) étant prévu avec au moins une trémie d'entretien (11), la trémie d'entretien (11) présentant au moins un orifice de regard (10) pour faciliter notamment le montage et le démontage et/ou l'entretien des appareils comme par exemple d'un agitateur (9),
la trémie d'entretien (11) étant agencée sur un toit de cuve (15) recouvrant l'espace intérieur du digesteur (22) de façon à rendre le toit hermétique, et au moins une tige de montage (9a) étant prévue pénétrant dans l'intérieur du digesteur (22) et notamment dans la masse de fermentation (23) et venant de la trémie d'entretien (11), cette tige étant agencée à une certaine distance de la paroi de cuve (12),
**caractérisées en ce**
**que** la trémie d'entretien (11) est fixée à un cadre de support (2) qui comprend au moins une zone de support (2a), des moyens de fixation (1) étant prévus sur la zone de support (2a), avec lesquels la zone de support (2a) peut être orientée et fixée en-dehors de l'espace intérieur du digesteur (21).
